# EUROPEAN PATENT SPECIFICATION

(11) **EP 1 546 090 B1**
(45) Date of publication and mention of the grant of the patent: **24.06.2009**
(21) Application number: 03798091.9
(22) Date of filing: 25.09.2003
(51) Int. Cl.: C07C 235/72, C07K 1/06, G01N 33/68, A61K 38/28, C07K 14/605, C07K 14/62

(54) **METHOD FOR PRODUCING ACYLATED PEPTIDES**
VERFAHREN ZUR HERSTELLUNG ACYLIERTER PEPTIDE
PROCEDE DE PRODUCTION DE PEPTIDES ACYLES

(30) Priority: 25.09.2002 DK 200201421
(43) Date of publication of application: 29.06.2005
(73) Proprietor: NOVO NORDISK A/S, 2880 Bagsvaerd (DK)
(72) Inventor: DÜNWEBER, Dorte, Lunöe, DK-2830 Virum (DK); JENSEN, Inge, Holm, DK-2765 Smörum (DK); HANSEN, Louis, Brammer, DK-3500 Värlöse (DK)
(86) International application number: PCT/DK2003/000629
(87) International publication number: WO 2004/029077

(56) References cited:
- EP-A- 1 227 107
- WO-A-00/55119
- WO-A-95/07931
- WO-A-98/02460
- WO-A-98/08871
- WO-A-98/08872
- WO-A-99/43708
- US-A- 3 950 517
- US-B1- 6 451 974
- KANJI MEGURO ET AL: "Studies on Antidiabetic Agents. VII.1) Synthesis and Hypoglycemic Activity of 4-Oxazoleacetic Acid Derivatives2)" CHEM. PHARM. BULL., vol. 34, no. 7, 1986, pages 2840-2851, XP002266282
- RIORDAN J F ET AL: "ACETYLATION" METHODS IN ENZYMOLOGY, vol. 25, no. PARTB, 1972, pages 494-499, XP002266297 Academic Press Inc, San Diego, CA, US

## Description

### FIELD OF THE INVENTION

The present invention relates to methods for acylating peptides and proteins. More specifically, the invention relates to a method of introducing one or more acyl groups into a peptide or a protein.

### BACKGROUND OF THE INVENTION

A large number of peptides have been approved for use in medical practice, and the peptides may be produced in suitable host cells by recombinant DNA technology or they may be produced synthetically by well established peptide synthesis technology. However, native peptides as well as analogues thereof tend to exhibit high clearance rates which are unacceptable for many clinical indication where a high plasma concentration of the peptide is required over a prolonged period of time. Examples of peptides which in their native form have a high clearance are: ACTH, corticotropin-releasing factor, angiotensin, calcitonin, insulin, glucagon, glucagon-like peptide-1 (GLP-1), glucagon-like peptide-2 (GLP-2), insulin-like growth factor-1, insulin-like growth factor-2, gastric inhibitory peptide, growth hormone-releasing factor, pituitary adenylate cyclase activating peptide, secretin, enterogastrin, somatostatin, somatotropin, somatomedin, parathyroid hormone, thrombopoietin, erythropoietin, hypothalamic releasing factors, prolactin, thyroid stimulating hormones, endorphins, enkephalins, vasopressin, oxytocin, opioids and analogues thereof, superoxide dismutase, interferon, asparaginase, arginase, arginine deaminase, adenosine deaminase and ribonuclease.

A variety of derivatizations of peptides and peptide analogs have been found to influence the clearance rate of the peptides in a favourable direction. One such derivatization is the introduction of a lipophilic acyl group into the therapeutic peptide causing a desirable protracted profile of action relative to the non-acylated peptide. Hence, less frequent administration of the therapeutic protein improves the patients compliance to the prescribed therapy, and it reduces the amount of peptide to be administered. This has been described and demonstrated in WO98/08871, which i.a. discloses acylation of GLP-1 and analogs thereof, in WO98/08872, which i.a. discloses acylation of GLP-2 and analogs thereof, and
WO99/43708, which i.a. discloses acylation of exendin and analogs thereof. Mono- or dipeptide spacers such as aspartic acid and glutamic acid, between the peptide and the acyl-group was demonstrated to be desirable. Spacers including a free carboxylic acid group must be protected before acylation and subsequently deprotected.
WO 98 02460 and EP 1227107 disclose the acylation of ε-amino groups of human insulin and WO 95 07931 and US 3 950 517 disclose acylations at various amino acids in insulins. WO00/55119 and US 6 451 974 disclose a method for acylating peptides (e.g. GLP-1) and novel acylating agents in e.g. a mixture of aprotic polar solvent and water wherein the ratio between the aprotic polar solvent and water is between 1:10 and 10:1.
Kanji Meguro et al. disclose the synthesis of 4-oxazolealkanoic acid derivatives such as various 3-acyl-3-acylaminopropionates (Chem. Pharm. Bull. Vol.34, No.7 (1986) pp.2840-2851) and J. F. Riordan and B. L. Vallee disclose some commonly used acetylation methods (Meth. Enzym., Volume 25, 1972, Pages 494-499).

In order for therapeutic peptides to be economically viable the cost of producing the peptides as well as the therapeutic dosage of the peptide are pivotal. A major cost during production of therapeutic peptides is the purification steps required to separate the target protein from impurities which are closely related to the target protein, e.g. isomers, desamido forms etc. These purification steps are usually performed by chromatography implying expensive chromatography matrices and solvents as well as reduced overall yield.
It is the aim of the present invention to provide an efficient and economic method for the introduction of lipophilic groups into peptides via α-amino-α,ω-dicarboxylic acid spacers. The method is more specific, and thus results in higher yields and reduced formation of closely related impurities. A significant reduction of the cost of producing the acylated peptides are achieved. Less expensive acylated peptides are highly desirable for maximizing the number of patients for whom the treatment is available as well as for exploiting the advantages of alternative delivery routes which have lower bioavailability than subcutaneous injection, e.g. transdermal and pulmonal delivery.

### SUMMARY OF THE INVENTION

The present invention provides a method for acylating one or more amino groups of a peptide or protein, the method comprising the steps :
a) reacting the peptide having at least one free amino group with an acylating agent of the general formula I 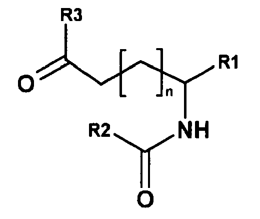 wherein
   n is 0-8;
   R¹ is COOR⁴;
   R² is a lipophilic moiety;
   R³ together with the carboxyl group to which R³ is attached designate a reactive ester or a reactive N-hydroxy imide ester; and
   R⁴ is selected from hydrogen, C₁₋₁₂-alkyl and benzyl,
   under basic conditions in an aqueous mixture;
b) if R⁴ is not hydrogen, saponifying the acylated peptide ester group (COOR⁴) under basic conditions;
c) isolating the N-acylated peptide,
characterised by said aqueous mixture in step a) containing less than 8 %w/w aprotic polar solvent.

In one embodiment of the method, said reaction in step a) takes place in an aqueous mixture containing less than 5 %w/w aprotic polar solvent and preferable less than 3 %w/w aprotic polar solvent.
In another embodiment of the method, the acylating agent is added to the reaction mixture as a solid.
In another embodiment of the method, the acylating agent is added to the reaction mixture as a solution in a aprotic polar solvent which is stabilized by adding an acid.

### DESCRIPTION OF THE INVENTION

### Peptides and proteins

The present invention is useful for the introduction of lipophilic acyl groups into any peptide (or protein) in order to reduce the *in vivo* clearance rate. Examples of such peptides and proteins are ACTH, corticotropin-releasing factor, angiotensin, calcitonin, exendin and analogues thereof, insulin and analogues thereof, glucagon and analogues thereof, glucagon-like peptide-1 and analogues thereof, glucagon-like peptide-2 and analogues thereof, insulin-like growth factor-1, insulin-like growth factor-2, gastric inhibitory peptide, growth hormone-releasing factor, pituitary adenylate cyclase activating peptide, secretin, enterogastrin, somatostatin, somatotropin, somatomedin, parathyroid hormone, thrombopoietin, erythropoietin, hypothalamic releasing factors, prolactin, thyroid stimulating hormones, endorphins, enkephalins, vasopressin, oxytocin, opiods and analogues thereof, superoxide dismutase, interferon, asparaginase, arginase, arginine deaminase, adenosine deaminase and ribonuclease.

It should be understood that the peptide (or protein) should carry at least one free amino group, such an amino group being the N-terminal amino group or a side chain amino group. The peptides or protein may comprise amino acids which are not encoded by the genetic code, such as D-amino acids, 3-hydroxyproline, ornithine and pentylglycine. Particularly interesting are amino groups of lysine and ornithine amino acid residues. The method is particular relevant for the N-acylation of the ε-amino group of lysine residues. It should also be understood that the peptide or protein in question may comprise two or more pendant amino groups which all may be N-acylated according to the present invention.

The present invention is especially suitable for the acylation of GLP-1 and analogues thereof. Examples of GLP-1 and analogues which can be N-acylated according to the present invention are GLP-1 and truncated analogues, such as Arg²⁶-GLP-1(7-37); Arg³⁴-GLP-1(7-37); Lys³⁶-GLP-1(7-37); Arg^{26,34}Lys³⁶-GLP-1(7-37); Arg^{26,34}Lys³⁸GLP-1 (7-38); Arg^{26,34}Lys³⁹-GLP-1(7-39); Arg^{26,34}Lys⁴⁰-GLP-1(7-40); Arg³⁴Lys³⁶-GLP-1(7-37); Arg²⁶Lys³⁹-GLP-1(7-39); Arg³⁴Lys⁴⁰-GLP-1(7-40); Arg^{26,34}Lys^{36,39}-GLP-1 (7-39); Arg^{26,34}Lys^{36,40}-GLP-1 (7-40); Gly⁸Arg²⁶-GLP-1 (7-37); Gly⁸Arg³⁴-GLP-1(7-37); Gly⁸Lys³⁶-GLP-1(7-37); Gly⁸Arg^{26,34}Lys³⁶-GLP-1(7-37); Gly⁸Arg^{26,34}Lys³⁹-GLP-1(7-39); Gly⁸Arg^{26,34}Lys⁴⁰-GLP-1 (7-40); Gly⁸Arg²⁶Lys³⁶-GLP-1(7-37); Gly⁸Arg³⁴Lys³⁶-GLP-1(7-37); Lys³⁶-GLP-1 (7-37); Arg^{26,34}Lys³⁶-GLP-1 (7-37); Arg^{26,34}-GLP-1 (7-37); Arg^{26,34}Lys⁴⁰-GLP-1(7-37); Arg²⁶Lys³⁶-GLP-1 (7-37); Arg³⁴Lys³⁶-GLP-1(7-37); Val⁸Arg²²-GLP-1 (7-37); Met⁸Arg²²-GLP-1 (7-37);Gly⁸His²²-GLP-1(7-37); Val⁸His²²-GLP-1(7-37); Met⁸His²²-GLP-1(7-37);His³⁷-GLP-1 (7-37); Gly⁸-GLP-1(7-37); Val⁸-GLP-1(7-37); Met⁸-GLP-1(7-37);Gly⁸Asp²²-GLP-1 (7-37); Val⁸Asp²²-GLP-1(7-37); Met⁸Asp²²-GLP-1 (7-37);Gly⁸Glu²²-GLP-1(7-37); Val⁸Glu²²-GLP-1(7-37); Met⁸Glu²²-GLP-1(7-37); Gly⁸Lys²²-GLP-1 (7-37); Val⁸Lys²²-GLP-1 (7-37); Met⁸Lys²²-GLP-1 (7-37); Gly⁸Arg²²-GLP-1 (7-37); Val⁸Lys²²His³⁷-GLP-1(7-37); Gly⁸Glu²²His³⁷-GLP-1 (7-37); Val⁸Glu²²His³⁷-GLP-1(7-37); Met⁸Glu²²His³⁷-GLP-1(7-37);Gly⁸Lys²² His³⁷-GLP-1 (7-37); Met⁸Lys²²His³⁷ -GLP-1 (7-37);Gly⁸Arg²²His³⁷-GLP-1(7-37); Val⁸Arg²²His³⁷-GLP-1(7-37); Met⁸Arg²²His³⁷-GLP-1(7-37); Gly⁸His²²His³⁷-GLP-1 (7-37); Val⁸His²²His³⁷-GLP-1(7-37); Met⁸His²²His³⁷-GLP-1 (7-37); Gly⁸His³⁷-GLP-1 (7-37); Val⁸His³⁷-GLP-1 (7-37); Met⁸His³⁷-GLP-1(7-37);Gly⁸Asp²² His³⁷-GLP-1 (7-37); Val⁸Asp²²His³⁷-GLP-1(7-37); Met⁸Asp²²His³⁷-GLP-1 (7-37); Arg²⁶-GLP-1 (7-36)-amide; Arg³⁴-GLP-1 (7-36)-amide; Lys³⁶-GLP-1 (7-36)-amide; Arg^{26,34}Lys³⁶-GLP-1 (7-36)-amide; Arg^{26,34}-GLP-1 (7-36)-amide; Arg^{26,34}Lys⁴⁰-GLP-1 (7-36)-amide; Arg²⁶Lys³⁶-GLP-1(7-36)-amide; Arg³⁴Lys³⁶-GLP-1 (7-36)-amide; Gly⁸-GLP-1(7-36)-amide; Val⁸-GLP-1(7-36)-amide; Met⁸-GLP-1 (7-36)-amide;Gly⁸Asp²²-GLP-1(7-36)-amide; Gly⁸Glu²²His³⁷-GLP-1(7-36)-amide; Val⁸Asp²²-GLP-1 (7-36)-amide; Met⁸Asp²²-GLP-1 (7-36)-amide;Gly⁸Glu²²-GLP-1(7-36)-amide; Val⁸Glu²²-GLP-1(7-36)-amide; Met⁸Glu²²-GLP-1 (7-36)-amide; Gly⁸Lys²²-GLP-1(7-36)-amide; Val⁸Lys²²-GLP-1(7-36)-amide; Met⁸Lys²²-GLP-1(7-36)-amide; Gly⁸His²²His³⁷-GLP-1(7-36)-amide; Gly⁸Arg²²-GLP-1 (7-36)-amide; Val⁸Arg²²-GLP-1 (7-36)-amide; Met⁸Arg²²-GLP-1 (7-36)-amide;Gly⁸His²²-GLP-1 (7-36)-amide; Val⁸His²²-GLP-1 (7-36)-amide; Met⁸His²²-GLP-1 (7-36)-amide;His³⁷-GLP-1 (7-36)-amide; Val⁸Arg²²His³⁷-GLP-1(7-36)-amide; Met⁸Arg²²His³⁷-GLP-1 (7-36)-amide; Gly⁸His³⁷-GLP-1 (7-36)-amide; Val⁸His³⁷-GLP-1(7-36)-amide; Met⁸His³⁷-GLP-1(7-36)-amide;Gly⁸Asp²² His³⁷-GLP-1 (7-36)-amide; Val⁸Asp²²His³⁷-GLP-1 (7-36)-amide; Met⁸Asp²²His³⁷-GLP-1(7-36)-amide; Val⁸Glu²²His³⁷-GLP-1(7-36)-amide; Met⁸Glu²²His³⁷-GLP-1(7-36)-amide;Gly⁸Lys²²His³⁷-GLP-1 (7-36)-amide; Val⁸Lys²²His³⁷-GLP-1(7-36)-amide; Met⁸Lys²²His³⁷-GLP-1 (7-36)-amide;Gly⁸Arg²²His³⁷-GLP-1 (7-36)-amide; Val⁸His²²His³⁷-GLP-1 (7-36)-amide; Met⁸His²²His³⁷-GLP-1 (7-36)-amide; Each of these GLP-1 analogues and truncated analogues constitutes alternative embodiments of the present invention.

The present invention is also especially suitable for the acylation of GLP-2 and analogues thereof. Examples of GLP-2 and analogues which can be N-acylated according to the present invention are GLP-2 analogues and truncated analogues, such as Lys²⁰GLP-2(1-33); Lys²⁰Arg³⁰GLP-2(1-33); Arg³⁰Lys³⁴GLP-2(1-34); Arg³⁰Lys³⁵GLP-2(1-35); Arg^{30,35}Lys²⁰GLP-2(1-35); and Arg³⁵GLP-2(1-35). Each of these GLP-2 analogues and truncated analogues constitutes alternative embodiments of the present invention.

The present invention is also especially suitable for the acylation of exendin-3 and exendin-4 and analogues thereof. Examples of exendin analogues which can be N-acylated according to the present invention are disclosed in e.g. WO99/43708. Each of these exendin analogues and truncated analogues constitutes alternative embodiments of the present invention.

The present invention is also particularly suited for the acylation of insulin and analogues thereof. Examples of insulin and analogues thereof which can be N-acylated according to the present invention are human insulin and des(B30)-human insulin.

In a further embodiment of the present invention the N-acylation takes place at the ε-amino group of lysine residues.

### Acylating agent

In the method according to the invention, a peptide (or protein) which has at least one free amino group is reacted with an acylating agent of the general formula I

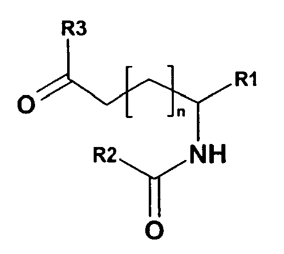

The integer n in formula I is preferably 0-8, in particular 0-6 corresponding, e.g., to aspartic acid, glutamic acid, etc. Preferably, n is 0-4 such as 0-2, e.g. 0 (aspartic acid) or 1 (glutamic acid). Each of these integers and ranges constitutes alternative embodiments of the present invention.

R¹ in formula I represents a free acid group (COOH) or an ester group (COOR⁴). In the cases where R¹ is an ester group, R⁴ is selected from groups which can be removed (as the corresponding alcohols) by hydrolysis under basic conditions. Examples of such groups are C₁₋₁₂-alkyl, e.g. methyl, ethyl, prop-1-yl, prop-2-yl, but-1-yl, but-2-yl, 2-methyl-prop-1-yl, 2-methyl-prop-2-yl (tert-butyl), hex-1-yl, etc., and benzyl. Each of these groups constitutes alternative embodiments of the present invention.

R² in formula I represents the lipophilic moiety to be incorporated into the peptide or protein. Such a lipophilic moiety is typically selected from C₃₋₃₉-alkyl, C₃₋₃₉-alkenyl, C₃₋₃₉-alkadienyl and steroidal residues. Specific examples of C₃₋₃₉-alkyl are heptyl, nonyl, undecanyl, tridecanyl, pentadecanyl, heptadecanyl, and nonadecanyl. Each of these lipophilic moieties constitutes alternative embodiments of the present invention.

The lipophilic substituent or moiety is characterised by having a solubility in water at 20°C in the range from about 0.1 mg/100 ml water to about 250 mg/100 ml water, preferable in the range from about 0.3 mg/100 ml water to about 75 mg/100 ml water. For instance, octanoic acid (C8) has a solubility in water at 20°C of 68 mg/100 ml, decanoic acid (C10) has a solubility in water at 20°C of 15 mg/100 ml, and octadecanoic acid (C18) has a solubility in water at 20°C of 0.3 mg/100 ml. Each of these lipophilic substituent ranges constitutes alternative embodiments of the present invention.

The terms "C₃₋₃₉-alkyl", "C₃₋₃₉-alkenyl" and "C₃₋₃₉-alkadienyl" is intended to cover straight chain and branched, preferably straight chain, saturated, mono-unsaturated and di-unsaturated, respectively, hydrocarbon radicals of 3-39 carbon atoms. Specific examples of C₃₋₃₉-alkyl are heptyl, nonyl, undecanyl, tridecanyl, pentadecanyl, heptadecanyl, and nonadecanyl.

When used herein, the term "steroidal residue" is intended to mean a lipophilic group which together with the carbonyl group to which R² is attached is derived from a steroide carboxylic acid, i.e. a tri-, tetra- and pentacyclic, full saturated or partially unsaturated C₁₆₋₃₆-hydrocarbon. Examples of such groups R²-C(=O)- are lithocholoyl, deoxycholoyl, and choloyl.

Among the lipophilic groups mentioned above, C₇₋₂₅-alkyl, C₇₋₂₅-alkenyl, C₇₋₂₅-alkadienyl and steroidal residues are especially relevant. Particularly interesting examples are heptyl, nonyl, undecanyl, tridecanyl, pentadecanyl, heptadecanyl, nonadecanyl, lithocholoyl, deoxycholoyl, and choloyl. Each of these lipophilic groups constitutes alternative embodiments of the present invention.

R³ in formula I together with the carboxyl group to which R³ is attached designate a reactive ester or a reactive N-hydroxy imide ester. Each of these esters constitutes alternative embodiments of the present invention. Reactive esters and reactive N-hydroxy imide esters are well known in the art of organic chemistry (especially in peptide chemistry) as functional groups which are used in acylation of amino, thio and hydroxy groups. Within the context of the present invention, the term "reactive ester or a reactive N-hydroxy imide ester" is intended to mean an ester functionalised form of a carboxylic acid group suitable for acylating an amine, preferably an primary amine. It should, thus, be understood, that selectivity for acylation of primary amines is preferred over acylating of hydroxy and thio groups. Reactive N-hydroxy imide esters are especially preferred.

Examples of reactive esters are 1-hydroxybenzotriazole esters and derivatives. A number of highly effective reagents, e.g. 2-(1H-benzotriazol-1yl)-1,1,3,3,-tetramethyluronium tetrafluoroborate, for the formation of such activated esters of carboxylic acids are known. Such reactive esters are typically formed *in situ* in the presence of a base, e.g. an organic base such as an trialkylamine.

Examples of the imide part of reactive N-hydroxy imide esters are those specifically described in EP 0511600 A2, page 3 to page 7. Especially interesting examples of imide parts among those are succinimide, phthalimide, etc. Each of these imide parts constitutes alternative embodiments of the present invention.

The reactive N-hydroxy imide esters of the formula I can be prepared as described in WO00/55119 and WO98/02460.

In the event where the acylating reagent of the formula I is to be used as the free α-carboxylic acid (R⁴ = hydrogen), a compound of the formula I where R⁴ is a group which can be removed selectively is converted to the corresponding compound where R⁴ is hydrogen. The carboxylic acid protecting group may be a benzyl group which can be removed by catalytic hydrogenation or an allyl group which can be selectively removed. A benzyl protecting group may be removed by catalytic hydrogenation in an aprotic polar solvent, e.g. in acetone, at room temperature by using palladium-on-carbon and hydrogen. The reaction may be performed in a closed vessel with an hydrogen atmosphere (typically 0.1-10 atm) under vigorous stirring. The reaction is typically completed within 0.5-12 hours depending on the quality of the palladium catalyst. Conventional work-up applies.

### Reaction conditions

The reaction between the acylating agent of the formula I and the peptide or protein is performed under basic conditions in an aqueous solution containing less than 8 %w/w of an aprotic polar solvent.
In one embodiment of the invention the reaction in step a) is performed in an aqueous solution containing from 1 %w/w to 8 %w/w of an aprotic polar solvent.

The acylating agent of the formula I is typically used in a slight excess relative to the number of amino groups of the peptide to be acylated. The ratio is typically 1:1 to 1:20 with an excess of the acylating agent, preferably 1:1.2 to 1:5, taking into account the number of amino groups in the peptide. The acylating agent may be added to the reaction mixture as a solid or it may be added to the reaction mixture as a solution. When the acylating agent is added as a solution it is dissolved in an aprotic polar solvent and preferably stabilized by adding an acid. Typically, the acid for the stabilization is a mineral acid, e.g. sulfuric acid.

It should be understood that the peptide may be fully N-acylated or only partially N-acylated depending on the amount of acylating agent used and the reaction conditions. It is preferred that the N-acylation is substantially stoichiometrical.

Aprotic polar solvents are solvents with moderately high dielectric constants which do not contain acidic hydrogen (see e.g. Morrison and Boyd, Organic Chemistry, 5^{th} ed. p 229). Typically, the aprotic polar solvent is selected from anhydrous tetrahydrofuran (THF), anhydrous dimethylformamide (DMF), acetone, dichloromethane, dimethylsulfoxide (DMSO), dioxane, dimethylacetamide, and N-methyl-2-pyrrolidone and mixtures thereof, among which dimethylformamide, dimethylsulfoxide, dimethylacetamide and N-methyl-2-pyrrolidone are preferred and N-methyl-2-pyrrolidone is especially preferred.

The temperature is typically kept in the range of -10-50 °C.

It is important that the pH value of the solvent mixture is in the range of 7-14, such as 9-13, preferably in the range of 10-12, in order for the reaction to proceed smoothly. The result with respect to yield and purity is normally optimal when the pH value of the solvent mixture is in the range of 10-12. The desired pH value is obtained by addition of alkalimetal hydroxides, e.g. sodium hydroxide and potassium hydroxide, and/or organic bases such as trialkylamines (e.g. triethylamine, N,N-diisopropylethylamine, etc.). It may also be advantageous to add a buffer which is suitable for keeping the pH near the starting value before the reaction starts. Examples of buffer which may be used for this purpose is phosphate buffer, borate buffer and the like.

As a typical example, the reaction in step (a) is performed using the protein and the acylating agent of the formula I in a 1:1 to 1:5 molar ratio. The peptide is typically pre-dissolved in water at -10-30°C such as 0-25°C and the pH is adjusted to the desired level using a alkalimetal hydroxide (e.g. sodium hydroxide or potassium hydroxide). The pH value may be further adjusted using acids, e.g. acetic acid, and bases, e.g. trialkylamine, but the temperature is preferably within the above range. Alternatively the peptide is pre-dissolved directly in an aqueous solution of an appropriate amount of the relevant acid or base. The acylating agent is subsequently added as a solid or as a solution in an aprotic polar solvent. The reaction is typically allowed to proceed to completion (can be monitored by HPLC) which is typically obtained within 0.2-4 hours, such as 0.2-1 hour, before addition of water and an acid, e.g. acetic acid, to pH 6.5-9.0. The product is typically isolated and purified by HPLC, or is precipitated by isoelectric pH, or is hydrolysed (step (b)) before purification.

When an acylating agent of the formula I where R⁴ is hydrogen is used, the N-acylated peptide or protein carrying lipophilic moieties and free carboxylic groups is obtained directly. Thus, the variant where R⁴ is hydrogen represents a preferred embodiment of the method of the present invention.

Alternatively, i.e. when the group R⁴ is C₁₋₁₂-alkyl or benzyl, the N-acylated peptide ester (or protein ester) is saponified under basic conditions so as to obtain the N-acylated peptide or N-acylated protein. Saponification is typically performed in a 0.01-4.0 M solution of an alkalimetal hydroxide, e.g. sodium or potassium hydroxide. The pH of the solution is typically 10-14. The reaction is typically allowed to proceed for 0.1-12 hours, preferably for 0.5-4 hours, at 0-40°C such as around room temperature. After reaction, the product is purified, e.g. by precipitation at isoelectric pH and/or by preparative HPLC. Thus, the variant where R⁴ is C_{1- 12}-alkyl or benzyl represents another preferred embodiment of the method of the present invention.

In one embodiment of the method, the acylating agent is added to the reaction mixture as a solid.

In one embodiment of the method, said reaction in step a) takes place in an aqueous mixture containing from 0 %w/w to 8 %w/w aprotic polar solvent, preferably from 0 %w/w to 5 %w/w aprotic polar solvent and even more preferable from 0 %w/w to 3 %w/w aprotic polar solvent.

In another embodiment of the method, said reaction in step a) takes place in the presence of an aprotic polar solvent, and said aprotic polar solvent is selected from the group consisting of N-methyl-2-pyrrolidone, tetrahydrofurane and dimethylsulfoxide.

In yet another embodiment of the method, all of the aprotic organic solvent is added to the reaction mixture as a solvent for the acylating agent.

In yet another embodiment of the method, the acylating agent is added to the reaction mixture as a solution which is stabilized by adding an acid.

In yet another embodiment of the method, said acid is added to the aprotic polar solvent in a concentration from 0.01 %w/w to 1 %w/w, preferably in a concentration from 0.05 %w/w to 0.5 %w/w.

In yet another embodiment of the method, said acid is selected from the group consisting of sulphuric acid, methanesulphonic acid and trifluoroacetic acid.

In yet another embodiment of the method, the reaction in step a) takes place in the absence of an aprotic polar solvent.

In yet another embodiment of the method, R⁴ in formula I is hydrogen.

In yet another embodiment of the method, R⁴ is selected from C₁₋₈-alkyl and benzyl.

In yet another embodiment of the method, R³ together with the carboxyl group to which R³ is attached designate a reactive N-hydroxy imide ester.

In yet another embodiment of the method, the acylated peptide ester is saponified at a pH value in the range of 10-14, preferably in the pH range from 9-13.
In yet another embodiment of the method, the acylated peptide ester is saponified at a pH value in the range of 9-14, such as in the pH range from 10-13.

In yet another embodiment of the method, pH of the reaction mixture in step a) is from pH 9 to pH 13, preferably from pH 10 to pH 12, or more preferable from pH 11.0 to pH 11.5.

In yet another embodiment of the method, the reaction mixture in step a) comprises a buffer which is suitable for maintaining a substantially constant pH during the reaction. In one embodiment of the method said buffer is a phosphate buffer, a borate buffer or a mixture thereof.

In yet another embodiment of the method, the temperature of the reaction mixture in step a) is in the range of 0-50 °C, preferably in the range from 5-40 °C and more preferable in the range from 10-30°C.

In yet another embodiment of the method, R² is selected from C₃₋₃₉-alkyl, C₃₋₃₉-alkenyl, C₃₋₃₉-alkadienyl and steroidal residues.

In yet another embodiment of the method, R²-C(=O)- is selected from the group consisting of lithocholoyl and hexadecanoyl.

In yet another embodiment of the method, said peptide used as starting material for step a) has a peptide purity of at least 80%, at least 90%, at least 93%, at least 95%, or at least 97% as determined by RP-HPLC.

In yet another embodiment of the method, said peptide is selected from the group consisting of GLP-1, exendin-4, GLP-2, glucagon, insulin, analogues thereof and derivatives of any of the foregoing.

In yet another embodiment of the method, said peptide is a GLP-1 agonist.

In yet another embodiment of the method, said peptide is selected from the group consisting of exendin-3, exendin-4, Arg³⁴-GLP-1(7-37), Gly⁸-GLP-1(7-36)-amide, Gly⁸-GLP-1 (7-37), Val⁸-GLP-1 (7-36)-amide, Val⁸-GLP-1 (7-37), Val⁸Asp²²-GLP-1 (7-36)-amide, Val⁸Asp²²-GLP-1 (7-37) , Val⁸Glu²²-GLP-1(7-36)-amide , Val⁸Glu²²-GLP-1 (7-37), Val⁸Lys²²-GLP-1(7-36)-amide, Val⁸Lys²²-GLP-1 (7-37), Val⁸Arg²²-GLP-1(7-36)-amide, Val⁸Arg²²-GLP-1 (7-37), Val⁸His²²-GLP-1 (7-36)-amide, Val⁸His²²-GLP-1 (7-37), des(B30)human insulin and derivatives thereof.

In yet another embodiment of the method, said peptide is selected from the group consisting of consisting of Val⁸Trp¹⁹Glu²² -GLP-1(7-37), Val⁸Glu²²Val²⁵-GLP-1 (7-37), Val⁸Tyr¹⁶Glu²²-GLP-1 (7-37), Val⁸Trp¹⁶Glu²²-GLP-1(7-37), Val⁸Leu¹⁶Glu²²-GLP-1(7-37), Val⁸Tyr¹⁸Glu²²-GLP-1 (7-37), Val⁸Glu²²His³⁷ -GLP-1 (7-37), Val⁸Glu²²Ile³³-GLP-1(7-37), Val⁸Trp¹⁶Glu²²Val²⁵Ile³³-GLP-1(7-37), Val⁸Trp¹⁶Glu²²Ile³³-GLP-1(7-37), Val⁸Glu²²Val²⁵Ile³³-GLP-1 (7-37), Val⁸Trp¹⁶Glu²²Val²⁵ -GLP-1(7-37) and analogues thereof.
In yet another embodiment of the method, said peptide is selected from HGEGTFTSDLSKQMEEEAVRLFIEWLKNGGPSSGAPPSKKKKKK-NH2 (ZP-10) and analogues thereof.

In another embodiment of the method, said peptide is not an insulin peptide, i.e. it is not insulin or an analogue thereof. In yet another embodiment of the method, said peptide comprises only one polypeptide chain. In yet another embodiment of the method, said peptide comprises two polypeptide chains which are covalently connected by at least one disulfide bond.

Another aspect of the present invention is the use of the acylation method for the preparation of a peptide derivative selected from the group consisting of Arg³⁴, Lys²⁶(N^{ε}-(γ-Glu(N^{α}-hexadecanoyl)))-GLP-1(7-37) and Lys^{B29}(N^{ε}-tetradecanoyl) des(B30) human insulin and Lys^{B29}(N^{ε}[N^{α}-lithocholoyl-Glu-OH]) des(B30) human insulin.

### EXAMPLES

Preparation of the acylation reagents is done as described in WO 00/55119.

In the examples final purification of the product was obtained by column chromatography.

### Example 1.

Arg-³⁴GLP-1₍₇₋₃₇₎ was expressed in yeast (*S. cerevisiae*) by conventional recombinant DNA technology, e.g. as described in WO 98/08871. Arg³⁴GLP-1 ₍₇₋₃₇₎ in the fermentation broth was then purified by conventional reversed phase chromatography and subsequently precipitated at the isoelectric pH of the peptide, i.e. at pH 5.4. The precipitate was isolated by centrifugation and frozen.
Arg³⁴-GLP-1⁷⁻³⁷ (1.47 g of frozen iso-precipitated peptide material, approx. 0.10 mmol) was dissolved in 0.1 mol/kg triethylamine (23 ml) at 10-15°C. The pH of the solution was 11.6. N-hexadecanoylglutamic acid γ-N-hydroxysuccinimide ester (63.7 mg, 0.13 mmol) was added. After 20 minutes at room temperature water (42 ml) was added, and the pH was adjusted to 8.0 by addition of 1.0 M acetic acid.

Yield: By analytical RP-HPLC the reaction mixture was shown to contain 84% (by area) of Arg³⁴Lys²⁶-[N-ε-(γ-Glu(N-hexadecanoyl))]-GLP-1⁷⁻³⁷ and 0.5% (by area) of Arg³⁴Lys²⁶-[N-ε-(α-Glu(N-hexadecanoyl))]-GLP-1⁷⁻³⁷.

### Example 2.

Arg³⁴GLP-1₍₇₋₃₇₎ was expressed in yeast (*S. cerevisiae*) by conventional recombinant DNA technology, e.g. as described in WO 98/08871. Arg³⁴GLP-1₍₇₋₃₇₎ in the fermentation broth was then purified by conventional reversed phase chromatography and subsequently precipitated at the isoelectric pH of the peptide, i.e. at pH 5.4. The precipitate was isolated by centrifugation and frozen.

Arg³⁴-GLP-1⁷⁻³⁷ (1.57 g of frozen iso-precipitated peptide material, approx. 0.14 mmol) was dissolved in 0.1 mol/kg triethylamine (23 ml) at 10-15°C. The pH of the solution was adjusted to 11.5 by the addition of triethylamine. 1.7 mL of N-hexadecanoylglutamic acid γ-N-hydroxysuccinimide ester (92.1 mg, 0.19 mmol) dissolved in N-methyl-2-pyrrolidone containing 0.105 %w/w 1 M H2SO₄ was added. After 20 minutes at room temperature water (42 ml) was added, and the pH was adjusted to 8.0 by addition of 1.0 M acetic acid.

Yield: By analytical RP-HPLC the reaction mixture was shown to contain 83% (by area) of Arg³⁴Lys²⁶-[N-ε-(γ-Glu(N-hexadecanoyl))]-GLP-1⁷⁻³⁷ and 0.4% (by area) of Arg³⁴Lys²⁶-[N-ε-(α-Glu(N-hexadecanoyl))]-GLP-1⁷⁻³⁷.

### Example 3.

Arg³⁴GLP-1₍₇₋₃₇₎ was expressed in yeast (*S. cerevisiae*) by conventional recombinant DNA technology, e.g. as described in WO 98/08871. Arg³⁴GLP-1₍₇₋₃₇₎ in the fermentation broth was then purified by conventional reversed phase chromatography and subsequently precipitated at the isoelectric pH of the peptide, i.e. at pH 5.4. The precipitate was isolated by centrifugation and frozen.
Arg³⁴-GLP-1⁷⁻³⁷ (1.53 g of frozen iso-precipitated peptide material, approx. 0.13 mmol) was dissolved in 0.05 mol/kg triethylamine (25 ml) at room temperature. The pH of the solution was 10.9. 1.8 ml of N-hexadecanoylglutamic acid γ-N-hydroxysuccinimide ester (94.2 mg, 0.19 mmol) dissolved in N-methyl-2-pyrrolidone without H₂SO₄ was added. After 30 minutes at room temperature water (48 ml) was added, and the pH was adjusted to 8.0 by addition of 1.0 M acetic acid.

Yield: By analytical RP-HPLC the reaction mixture was shown to contain 75% (by area) of Arg³⁴Lys²⁶ -[N-ε-(γ-Glu(N-hexadecanoyl))]-GLP-1⁷⁻³⁷ and 4.0% (by area) of Arg³⁴Lys²⁶-[N-ε-(α-Glu(N-hexadecanoyl))]-GLP-1⁷⁻³⁷.

### Example 4 - reference example with aprotic polar solvent conc. > 10 %w/w.

Arg³⁴GLP-1₍₇₋₃₇₎ was expressed in yeast (*S. cerevisiae*) by conventional recombinant DNA technology, e.g. as described in WO 98/08871. Arg³⁴GLP-1₍₇₋₃₇₎ in the fermentation broth was then purified by conventional reversed phase chromatography and subsequently precipitated at the isoelectric pH of the peptide, i.e. at pH 5.4. The precipitate was isolated by centrifugation and frozen.
Arg³⁴-GLP-1⁷⁻³⁷ (1.57 g of frozen iso-precipitated peptide material, approx. 0.14 mmol) was dissolved in 0.1 mol/kg triethylamine (23 ml) at 10-15°C. N-methyl-2-pyrrolidone (6.8 ml) was added and the pH of the solution was adjusted to 11.5 by the addition of triethylamine. Then N-hexadecanoylglutamic acid γ-N-hydroxysuccinimide ester (92.1 mg, 0.19 mmol) dissolved in 1.7 ml N-methyl-2-pyrrolidone containing 0.105 %w/w 1 M H₂SO₄ was added. After 20 minutes at room temperature water (54 ml) was added, and the pH was adjusted to 8.0 by addition of 1.0 M acetic acid.

Yield: By analytical RP-HPLC the reaction mixture was shown to contain 87% (by area) of Arg³⁴Lys²⁶-[N-ε-(γ-Glu(N-hexadecanoyl))]-GLP-1⁷⁻³⁷ and 0.5% (by area) of Arg³⁴Lys²⁶-[N-ε-(α-Glu(N-hexadecanoyl))]-GLP-1⁷⁻³⁷.

### Example 5 - reference example with aprotic polar solvent conc. > 10 %w/w.

Arg³⁴GLP-1₍₇₋₃₇₎ was expressed in yeast (*S. cerevisiae*) by conventional recombinant DNA technology, e.g. as described in WO 98/08871. Arg³⁴GLP-1₍₇₋₃₇₎ in the fermentation broth was then purified by conventional reversed phase chromatography and subsequently precipitated at the isoelectric pH of the peptide, i.e. at pH 5.4. The precipitate was isolated by centrifugation and frozen.
Arg³⁴-GLP-1⁷⁻³⁷ (1.51 g of frozen iso-precipitated peptide material, approx. 0.13 mmol) was dissolved in 0.1 mol/kg triethylamine (20 ml) and N-methyl-2-pyrrolidone (100 ml) at 10-15°C. Then N-hexadecanoylglutamic acid γ-N-hydroxysuccinimide ester (74 mg, 0.15 mmol) dissolved in 1.4 ml N-methyl-2-pyrrolidone containing 0.105 %w/w 1 M H₂SO₄ was added. After 45 minutes at room temperature water (206 ml) was added, and the pH was adjusted to 8 by addition of 1.0 M acetic acid.

Yield: By analytical RP-HPLC the reaction mixture was shown to contain 57% (by area) of Arg³⁴Lys²⁶-[N-ε-(γ-Glu(N-hexadecanoyl))]-GLP-1⁷⁻³⁷ and 14% (by area) of Arg³⁴Lys²⁶-[N-ε-(α-Glu(N-hexadecanoyl))]-GLP-1⁷⁻³⁷.

### Example 6.

In table 1 the experimental conditions used for acylating Arg³⁴-GLP-1⁷⁻³⁷ in the experiments of example 1-5 are summarised and the resulting contents of the impurity Arg³⁴Lys²⁶-[N-ε-(α-Glu(N-hexadecanoyl))]-GLP-1⁷⁻³⁷ (abbreviated α-glu) are listed. Without the addition of H₂SO₄ as stabilizer it is clear that lower concentrations of aprotic polar solvent is effective in reducing the amount of the α-glu impurity. When the stabilizer is added the formation of significant amounts of the α-glu impurity start at significantly higher concentrations of aprotic polar solvent, i.e. above the concentration 28 %w/w.

**Table 1. The contents of aprotic polar solvent in the reaction mixture when acylating GLP-1 peptides and the resulting fraction of the impurity Arg³⁴Lys²⁶-[N-ε-(α-Glu(N-hexadecanoyl))]-GLP-1⁷⁻³⁷ (α-glu). (Data from Examples 1-5).**

| Example # | Aqueous peptide solution (mL) | NMP added (mL) | NMP contents (%w/w) | H₂SO₄ added | α-glu |
|---|---|---|---|---|---|
| 1 | 23 | 0 | 0% | - | 0.5% |
| 2 | 23 | 1.7 | 7% | + | 0.4% |
| 3 | 25 | 1.8 | 7% | - | 4% |
| 4 | 23 | 6.8 + 1.7 | 28% | + | 0.5% |
| 5 | 20 | 100 + 1.4 | 84% | + | 14% |

### Example 7.

Sodium-crystallized des(B30)-human insulin (1.74 g of frozen peptide material isolated by isoelectric precipitation, approx. 0.088 mmol) was dissolved in 0.1 mol/kg triethylamine (10.85 ml) at room temperature. The pH of the solution was 10.9. Then, methyl (2S)-5-[(2,5-dioxo-1-pyrrolidinyl)oxy]-2-{[(3a,5b)-3-hydroxy-9-methyl-24-oxocholan-24-yl]amino}-5-oxopentanoate (55.1 mg, 0.089 mmol) dissolved in N-methyl-2-pyrrolidone (1.25 ml) was added. After 30 minutes at room temperature water (5°C, 85 ml) was added.

Yield: By analytical RP-HPLC the reaction mixture was shown to contain 34% (by area) of Nε-(lithocholoyl-γ-glutamyl)-Lys^{B29}-des(B30)-human insulin.

### Example 8.

Arg³⁴GLP-1 ₍₇₋₃₇₎ was expressed in yeast (*S*.*cerevisiae*) by conventional recombinant DNA technology, e.g. as described in WO 98/08871. Arg³⁴GLP-1₍₇₋₃₇₎ in the fermentation broth was then purified by conventional reversed phase chromatography and subsequently precipitated at the isoelectric pH of the peptide, i.e. at pH 5.4. The precipitate was isolated by centrifugation and frozen.
The isoelectric precipitate containing Arg³⁴GLP-1₍₇₋₃₇₎ (5.70 g of frozen precipitated peptide material, approx. 0.38 mmol) was dissolved in 0.1 M disodiumhydrogenphosphate dihydrate solution (170 mL, pH adjusted to 11.35 with 1 M NaOH) at 15°C. The pH of the solution was re-adjusted to 11.4 by the addition of 1 M NaOH. 3 mL of N-hexadecanoylglutamic acid γ-N-hydroxysuccinimide ester (248.1 mg, 0.51 mmol) dissolved in N-methyl-pyrrolidone containing 0.105% w/w 1 M H₂SO₄ was added dropwise during 8 min.

Samples were drawn out during 21.5 hours of reaction at 15°C and added 0.1 M sodiumdihydrogenphosphate dihydrate solution containing 1 mg/mL glycine, pH 7.5. The final reaction mixture was diluted with water (300 mL) and the pH was adjusted to 8.0 by addition of glacial acid.

Yield: By analytical RP-HPLC the reaction mixture was shown to contain 78-80% (by area) of Arg³⁴Lys²⁶-[N-ε-(γ-Glu(N-hexadecanoyl))]-GLP-1⁷⁻³⁷ and 0.24-1.67% (by area) of Arg³⁴Lys²⁶-[N-ε-(α-Glu(N-hexadecanoyl))]-GLP-1⁷⁻³⁷.

**Table 2. The content of Arg³⁴Lys²⁶-[N-ε-(γ-Glu(N-hexadecanoyl))]-GLP-1⁷⁻³⁷ (γ-Glu) and Arg³⁴Lys²⁶-[N-ε-(α-Glu(N-hexadecanoyl))]-GLP-1⁷⁻³⁷ (α-Glu) vs. time of reaction. (Data from Example 8).**

| Time | γ-Glu | α-Glu |
|---|---|---|
| (hrs) | (% by area) | (% by area) |
| 0.17 | 78.01 | 0.24 |
| 1 | 79.35 | 0.31 |
| 2 | 79.45 | 0.36 |
| 4 | 80.49 | 0.45 |
| 21.5 | 80.27 | 1.67 |

## Claims

1. A method for producing an N-acylated peptide, said method comprising the steps:
a) reacting the peptide having at least one free amino group with an acylating agent of the general formula I 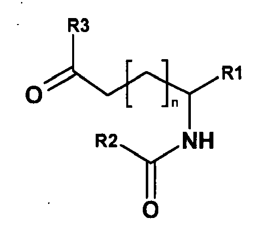 wherein
n is 0-8;
R¹ is COOR⁴;
R² is a lipophilic moiety;
R³ together with the carboxyl group to which R³ is attached designate a reactive ester or a reactive N-hydroxy imide ester; and
R⁴ is selected from hydrogen, C₁₋₁₂-alkyl and benzyl,
under basic conditions in an aqueous mixture;
b) if R⁴ is not hydrogen, saponifying the acylated peptide ester group (COOR⁴) under basic conditions;
c) isolating the N-acylated peptide,
**characterised by** said aqueous mixture in step a) containing less than 8 %w/w aprotic polar solvent.

2. The method according to claim 1, wherein said reaction in step a) takes place in an aqueous mixture containing less than 5 %w/w aprotic polar solvent, preferably less than 3 %w/w aprotic polar solvent.

3. The method according to any one of claims 1-2, wherein the acylating agent is added to the reaction mixture as a solid.

4. The method according to any one of claims 1-2, wherein said reaction in step a) takes place in the presence of an aprotic polar solvent, and said aprotic polar solvent is selected from the group consisting of N-methyl-2-pyrrolidone, tetrahydrofurane and dimethylsulfoxide.

5. The method according to claim 4, wherein all of the aprotic organic solvent is added to the reaction mixture as a solvent for the acylating agent.

6. The method according to any one of claims 4-5, wherein the acylating agent is added to the reaction mixture as a solution which is stabilized by adding an acid.

7. The method according to claim 6, wherein said acid is added to the aprotic polar solvent in a concentration from 0.01 %w/w to 1 %w/w, preferably in a concentration from 0.05 %w/w to 0.5 %w/w.

8. The method according to any one of claims 6-7, wherein said acid is selected from the group consisting of sulphuric acid, methanesulphonic acid and trifluoroacetic acid.

9. The method according to any one of claims 1-3, wherein the reaction in step a) takes place in the absence of an aprotic polar solvent.

10. The method according to any one of claims 1-9, wherein R⁴ is hydrogen.

11. The method according to any one of claims 1-9, wherein R⁴ is selected from C₁₋₈-alkyl and benzyl.

12. The method according to any of claims 1-11, wherein R³ together with the carboxyl group to which R³ is attached designate a reactive N-hydroxy imide ester.

13. The method according to any one of claims 1-9, wherein the acylated peptide ester is saponified at a pH value in the range of 10-14, preferably in the pH range from 9-13.

14. The method according to any one of claims 1-13, wherein pH of the reaction mixture in step a) is from pH 9 to pH 13, preferably from pH 10 to pH 12, or more preferable from pH 11.0 to pH 11.5.

15. The method according to any of claims 1-14, wherein the temperature of the reaction mixture in step a) is in the range of 0-50 °C, preferably in the range from 5-40 °C and more preferable in the range from 10-30 °C.

16. The method according to any of the preceding claims, wherein R² is selected from C₃₋₃₉-alkyl, C₃₋₃₉-alkenyl, C₃₋₃₉-alkadienyl and steroidal residues.

17. The method according to claim 16, wherein R²-C(=O)- is selected from the group consisting of lithocholoyl and hexadecanoyl.

18. The method according to any of the preceding claims, wherein said peptide used as starting material for step a) has a peptide purity of at least 80%, at least 90%, at least 93%, at least 95%, or at least 97% as determined by RP-HPLC.

19. The method according to any of the preceding claims, wherein said peptide is selected from the group consisting of GLP-1, exendin-4, GLP-2, glucagon and insulin.

20. The method according to any of the claims 1-18, wherein said peptide is an analogue of GLP-1 which is selected from the group consisting of: Arg²⁶-GLP-1(7-37); Arg³⁴-GLP-1(7-37); Lys³⁶-GLP-1(7-37); Arg^{26,34}Lys³⁶-GLP-1 (7-37); Arg^{26,34}Lys³⁸GLP-1(7-38); Arg^{26,34}Lys³⁹-GLP-1(7-39); Arg^{26,34}Lys⁴⁰-GLP-1(7-40); Arg³⁴Lys³⁶-GLP-1(7-37); Arg²⁶Lys³⁹-GLP-1(7-39); Arg³⁴Lys⁴⁰-GLP-1 (7-40); Arg^{26,34}Lys^{36,39}-GLP-1(7-39); Arg^{26,34}Lys^{36,40}-GLP-1(7-40); Gly⁸Arg²⁶-GLP-1(7-37); Gly⁸Arg³⁴-GLP-1(7-37); Gly⁸Lys³⁶-GLP-1(7-37); Gly⁸Arg^{26,34}Lys³⁶-GLP-1 (7-37); Gly⁸Arg^{26,34}Lys³⁹-GLP-1(7-39); Gly⁸Arg^{26,34}Lys⁴⁰-GLP-1 (7-40); Gly⁸Arg²⁶Lys³⁶-GLP-1(7-37); Gly⁸Arg³⁴Lys³⁶-GLP-1(7-37); Lys³⁶-GLP-1(7-37); Arg^{26,34}Lys³⁶-GLP-1(7-37); Arg^{26,34}-GLP-1(7-37); Arg^{26,34}Lys⁴⁰-GLP-1(7-37); Arg²⁶Lys³⁶-GLP-1(7-37); Arg³⁴Lys³⁶-GLP-1(7-37); Val⁸Arg²²-GLP-1(7-37); Met⁸Arg²²-GLP-1(7-37);Gly⁸His²²-GLP-1(7-37); Val⁸His²²-GLP-1(7-37); Met⁸His²²-GLP-1(7-37);His³⁷-GLP-1 (7-37); Gly⁸-GLP-1(7-37); Val⁸-GLP-1 (7-37); Met⁸-GLP-1 (7-37);Gly⁸Asp²²-GLP-1 (7-37); Val⁸Asp²²-GLP-1 (7-37); Met⁸Asp²²-GLP-1(7-37);Gly⁸Glu²²-GLP-1 (7-37); Val⁸Glu²²-GLP-1 (7-37); Met⁸Glu²²-GLP-1(7-37); Gly⁸Lys²²-GLP-1(7-37); Val⁸Lys²²-GLP-1 (7-37); Met⁸lys²²-GLP-1 (7-37); Gly⁸Arg²²-GLP-1(7-37); Val⁸Lys²²His³⁷-GLP-1(7-37); Gly⁸Glu²²His³⁷-GLP-1 (7-37); Val⁸Glu²²His³⁷-GLP-1(7-37); Met⁸Glu²²His³⁷-GLP-1(7-37);Gly⁸Lys²² His³⁷-GLP-1 (7-37); Met⁸Lys²²His³⁷-GLP-1(7-37);Gly⁸Arg²²His³⁷-GLP-1(7-37); Val⁸Arg²²His³⁷-GLP-1 (7-37); Met⁸Arg²²His³⁷-GLP-1(7-37); Gly⁸His²²His³⁷-GLP-1(7-37); Val⁸His²²His³⁷-GLP-1 (7-37); Met⁸His²²His³⁷-GLP-1 (7-37); Gly⁸His³⁷-GLP-1 (7-37); Val⁸His³⁷-GLP-1 (7-37); Met⁸His³⁷-GLP-1 (7-37);Gly⁸Asp²² His³⁷-GLP-1 (7-37); Val⁸Asp²²His³-GLP-1 (7-37); Met⁸Asp²²His³⁷-GLP-1 (7-37); Arg²⁶-GLP-1 (7-36)-amide; Arg³⁴ -GLP-1 (7-36)-amide; Lys³⁶-GLP-1 (7-36)-amide; Arg^{26,34}Lys³⁶-GLP-1 (7-36)-amide; Arg^{26,34}-GLP-1 (7-36)-amide; Arg^{26,34}Lys⁴⁰-GLP-1 (7-36)-amide; Arg²⁶Lys³⁶-GLP-1 (7-36)-amide; Arg³⁴Lys³⁶-GLP-1 (7-36)-amide; Gly⁸-GLP-1 (7-36)-amide; Val⁸-GLP-1 (7-36)-amide; Met⁸-GLP-1(7-36)-amide;Gly⁸ Asp²²-GLP-1 (7-36)-amide; Gly⁸Glu²²His³⁷-GLP-1 (7-36)-amide; Val⁸Asp²²-GLP-1(7-36)-amide; Met⁸Asp²²-GLP-1 (7-36)-amide;Gly⁸Glu²²-GLP-1 (7-36)-amide; Val⁸Glu²² -GLP-1 (7-36)-amide; Met⁸Glu²²-GLP-1 (7-36)-amide; Gly⁸Lys²² GLP-1 (7-36)-amide; Val⁸lys²2 -GlP-1 (7-36)-amide; Met⁸Lys²²-GLP-1 (7-36)-amide; Gly⁸His²²His³⁷-GLP-1 (7-36)-amide; Gly⁸Arg²²-GlP-1(7-36)-amide; Val⁸Arg²²-GLP-1 (7-36)-amide; Met⁸Arg²²-GLP-1 (7-36)-amide;Gly8His²²-GLP-1 (7-36)-amide; Val⁸His²²-GlP-1 (7-36)-amide; Met⁸His²²-GLP-1(7-36)-amide;His³⁷-GLP-1(7-36)-amide; Val⁸Arg²²His³⁷-GLP-1 (7-36)-amide; Met⁸Arg²²His³⁷-GLP-1 (7-36)-amide; Gly⁸His³⁷-GLP-1 (7-36)-amide; Val⁸His³⁷-GLP-1 (7-36)-amide; Met⁸His³⁷-GLP-1 (7-36)-amide;Gly⁸Asp²² His³⁷-GLP-1 (7-36)-amide; Val⁸Asp²²His⁷-GLP-1 (7-36)-amide; Met⁸Asp²²His³⁷-GLP-1 (7-36)-amide; Val⁸Glu²²His³⁷-GLP-1(7-36)-amide; Met⁸Glu²²His³⁷-GLP-1 (7-36)-amide;Gly⁸Glys²² His³⁷-GLP-1 (7-36)-amide; Val⁸Lys²²His³⁷-GLP-1(7-36)-amide; Met⁸Lys²²His³⁷-GLP-1 (7-36)-amide;Gly⁸Arg²²His³⁷-GLP-1 (7-36)-amide; Val⁸His²²His³⁷-GLP-1(7-36)-amide; and Met⁸His²²His³⁷-GLP-1(7-36)-amide.

21. The method according to any of the claims 1-18, wherein said peptide is an analogue of GLP-2 which is selected from the group consisting of: Lys²⁰GLP-2(1-33); Lys²⁰Arg³⁰GLP-2(1-33); Arg³⁰Lys³⁴GLP-2(1-34); Arg³⁰Lys³⁵GLP-2(1-35); Arg^{30,35}Lys²⁰GLP-2(1-35); and Arg³⁵GLP-2(1-35).

22. The method according to any of the claims 1-18, wherein said peptide is an analogue of insulin which is des(B30)-human insulin.

23. The method according to any one of the preceding claims, wherein said peptide is a GLP-1 agonist.

24. The method according to any one of the preceding claims, wherein said peptide is selected from the group consisting of exendin-3, exendin-4, Arg³⁴-GLP-1 (7-37), Gly⁸-GLP-1 (7-36)-amide, Gly⁸-GLP-1 (7-37), Val⁸-GLP-1 (7-36)-amide, Val⁸-GLP-1 (7-37), Val⁸Asp²² -GLP-1 (7-36)-amide, Val⁸Asp²²-GLP-1 (7-37) , Val⁸Glu²²-GLP-1(7-36)-amide, Val⁸Glu²²-GLP-1(7-37), Val⁸Lys²²-GLP-1(7-36)-amide, Val⁸Lys²²-GLP-1 (7-37), Val⁸Arg²²-GLP-1 (7-36)-amide, Val⁸Arg²²-GLP-1 (7-37), Val⁸His²²-GLP-1 (7-36)-amide, Val⁸His²²-GLP-1 (7-37), and des(B30)human insulin.

25. The method according to claim 19, wherein said peptide is HGEGTFTSDLSKQMEEEAVRLFIEWLKNGGPSSGAPPSKKKKKK-NH2 (ZP-10).

26. The method according to any one of claims 1-14, wherein the reaction mixture in step a) comprises a buffer which is suitable for maintaining a substantially constant pH during the reaction.

27. The method according to any of the preceding claims, wherein said peptide is not an insulin peptide.

## Patentansprüche

1. Verfahren zur Herstellung eines N-acylierten Peptids, wobei das Verfahren die folgenden Schritte umfasst:
a) Umsetzen des mindestens eine freie Aminogruppe aufweisenden Peptids mit einem Acylierungsmittel der allgemeinen Formel I 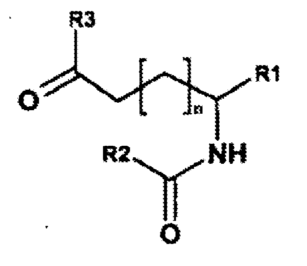 wobei
n für 0-8 steht;
R¹ für COOR⁴ steht;
R² für eine lipophile Einheit steht;
R³ zusammen mit der Carboxygruppe, an welche R³ angelagert ist einen reaktiven Ester oder einen reaktiven N-Hydroxyimidester bezeichnet; und
R⁴ ausgewählt ist aus Wasserstoff, C₁₋₁₂-Alkyl und Benzyl,
unter basischen Bedingungen in einem wässrigen Gemisch;
b) falls R⁴ nicht für Wasserstoff steht, Verseifen der acylierten Peptidestergruppe (COOR⁴) unter basischen Bedingungen;
c) Isolieren des N-acylierten Peptids,
**dadurch gekennzeichnet, dass** das wässrige Gemisch in Schritt a) weniger als 8 Gew.-% aprotisches polares Lösungsmittel enthält.

2. Verfahren nach Anspruch 1, wobei die Umsetzung in Schritt a) in einem wässrigen Gemisch stattfindet, das weniger als 5 Gew.-% aprotisches polares Lösungsmittel, vorzugsweise weniger als 3 Gew.-% aprotisches polares Lösungsmittel enthält.

3. Verfahren nach einem der Ansprüche 1-2, wobei das Acylierungsmittel dem Reaktionsgemisch als Feststoff zugesetzt wird.

4. Verfahren nach einem der Ansprüche 1-2, wobei die Umsetzung in Schritt a) in der Gegenwart eines aprotischen polaren Lösungsmittels stattfindet und das aprotische polare Lösungsmittel ausgewählt ist aus der Gruppe, bestehend aus N-Methyl-2-pyrrolidon, Tetrahydrofuran und Dimethylsulfoxid.

5. Verfahren nach Anspruch 4, wobei das ganze aprotische organische Lösungsmittel dem Reaktionsgemisch als Lösungsmittel für das Acylierungsmittel zugesetzt wird.

6. Verfahren nach einem der Ansprüche 4-5, wobei das Acylierungsmittel dem Reaktionsgemisch als eine durch Zugabe einer Säure stabilisierte Lösung zugesetzt wird.

7. Verfahren nach Anspruch 6, wobei die Säure dem aprotischen polaren Lösungsmittel in einer Konzentration von 0,01 Gew.-% bis 1 Gew.-%, vorzugsweise in einer Konzentration von 0,05 Gew.-% bis 0,5 Gew.-% zugesetzt wird.

8. Verfahren nach einem der Ansprüche 6-7, wobei die Säure ausgewählt ist aus der Gruppe, bestehend aus Schwefelsäure, Methansulfonsäure und Trifluoressigsäure.

9. Verfahren nach einem der Ansprüche 1-3, wobei die Umsetzung in Schritt a) in der Abwesenheit eines aprotischen polaren Lösungsmittels stattfindet.

10. Verfahren nach einem der Ansprüche 1-9, wobei R⁴ für Wasserstoff steht.

11. Verfahren nach einem der Ansprüche 1-9, wobei R⁴ ausgewählt ist aus C₁₋₈-Alkyl und Benzyl.

12. Verfahren nach einem der Ansprüche 1-11, wobei R³ zusammen mit der Carboxygruppe, an welche R³ angelagert ist, einen reaktiven N-Hydroxyimidester bezeichnet.

13. Verfahren nach einem der Ansprüche 1-9, wobei der acylierte Peptidester bei einem pH-Wert im Bereich von 10-14, vorzugsweise in dem pH-Bereich von 9-13 verseift wird.

14. Verfahren nach einem der Ansprüche 1-13, wobei der pH-Wert des Reaktionsgemischs in Schritt a) von pH 9 bis pH 13, vorzugsweise von pH 10 bis pH 12 oder stärker bevorzugt von pH 11,0 bis pH 11,5 beträgt.

15. Verfahren nach einem der Ansprüche 1-14, wobei die Temperatur des Reaktionsgemischs in Schritt a) im Bereich von 0-50°C, vorzugsweise im Bereich von 5-40°C und stärker bevorzugt im Bereich von 10-30°C liegt.

16. Verfahren nach einem der vorangehenden Ansprüche, wobei R² ausgewählt ist aus C₃₋₃₉-Alkyl-, C₃₋₃₉-Alkenyl-, C₃₋₃₉-Alkadienyl- und Steroidresten.

17. Verfahren nach Anspruch 16, wobei R²-C(=O)- ausgewählt ist aus der Gruppe, bestehend aus Lithocholoyl und Hexadecanoyl.

18. Verfahren nach einem der vorangehenden Ansprüche, wobei das für Schritt
a) als Ausgangsmaterial verwendete Peptid eine wie durch RP-HPLC bestimmte Peptidreinheit von mindestens 80%, mindestens 90%, mindestens 93%, mindestens 95% oder mindestens 97% aufweist.

19. Verfahren nach einem der vorangehenden Ansprüche, wobei das Peptid ausgewählt ist aus der Gruppe, bestehend aus GLP-1, Exendin-4, GLP-2, Glucagon und Insulin.

20. Verfahren nach einem der Ansprüche 1-18, wobei es sich bei dem Peptid um ein GLP1Analogon handelt, das ausgewählt ist aus der Gruppe, bestehend aus: Arg²⁶-GLP-1(7-37); Arg³⁴-GLP-1(7-37); Lys³⁶-GLP-1(7-37); Arg^{26,34}Lys³⁶-GLP-1(7-37); Arg^{26,34}Lys³⁸-GLP-1(7-38); Arg^{26,34}Lys³⁹-GLP-1(7-39); Arg^{26,34}Lys⁴⁰-GLP-1(7-40); Arg³⁴Lys³⁶-GLP-1(7-37); Arg²⁶Lys³⁹-GLP-1(7-39); Arg³⁴Lys⁴⁰-GLP-1(7-40); Arg^{26,34}Lys^{36,39}-GLP-1(7-39); Arg^{26,34}Lys^{36,40}-GLP-1(7-40); Gly⁸Arg²⁶-GLP-1(7-37); Gly⁸Arg³⁴-GLP-1(7-37); Gly⁸Lys³⁶-GLP-1(7-37); Gly⁸Arg^{26,34}Lys³⁶-GLP-1(7-37); Gly⁸Arg^{26,34}Lys³⁹-GLP-1(7-39); Gly⁸Arg^{26,34}Lys⁴⁰-GLP-1(7-40); Gly⁸Arg²⁶Lys³⁶-GLP-1(7-37); Gly⁸Arg³⁴Lys³⁶-GLP-1(7-37); Lys³⁶-GLP-1(7-37); Arg^{26,34}Lys³⁶-GLP-1(7-37); Arg^{26,34}-GLP-1(7-37); Arg^{26,34}Lys⁴⁰-GLP-1(7-37); Arg²⁶Lys³⁶-GLP-1(7-37); Arg³⁴Lys³⁶-GLP-1(7-37); Val⁸Arg²²-GLP-1(7-37); Met⁸Arg²²-GLP-1(7-37); Gly⁸His²²-GLP-1(7-37); Val⁸His²²-GLP-1(7-37); Met⁸His²²-GLP-1(7-37); His³⁷-GLP-1(7-37); Gly⁸-GLP-1(7-37); Val⁸-GLP-1(7-37); Met⁸-GLP-1(7-37); Gly⁸Asp²²-GLP-1(7-37); Val⁸Asp²²-GLP-1(7-37); Met⁸Asp²²-GLP-1(7-37); Gly⁸Glu²²-GLP-1(7-37); Val⁸Glu²²-GLP-1(7-37); Met⁸Glu²²-GLP-1(7-37); Gly⁸Lys²²-GLP-1(7-37); Val⁸Lys²²-GLP-1(7-37); Met⁸Lys²²-GLP-1(7-37); Gly⁸Arg²²-GLP-1(7-37); Val⁸Lys²²His³⁷-GLP-1(7-37); Gly⁸Glu²²His³⁷-GLP-1(7-37); Val⁸Glu²²His³⁷-GLP-1(7-37); Met⁸Glu²²His³⁷-GLP-1(7-37); Gly⁸Lys²²His³⁷-GLP-1(7-37); Met⁸Lys²²His³⁷-GLP-1(7-37); Gly⁸Arg²²His³⁷-GLP-1(7-37); Val⁸Arg²²His³⁷-GLP-1(7-37); Met⁸Arg²²His³⁷-GLP-1(7-37); Gly⁸His²²His³⁷-GLP-1(7-37); Val⁸His²²His³⁷-GLP-1(7-37); Met⁸His²²His³⁷-GLP-1(7-37); Gly⁸His³⁷-GLP-1(7-37); Val⁸His³⁷-GLP-1(7-37); Met⁸His³⁷-GLP-1(7-37); Gly⁸Asp²²His³⁷-GLP-1(7-37); Val⁸Asp²²His³⁷-GLP-1(7-37); Met⁸Asp²²His³⁷-GLP-1(7-37); Arg²⁶-GLP-1(7-36)amid; Arg³⁴-GLP-1(7-36)amid; Lys³⁶-GLP-1(7-36)amid; Arg^{26,34}Lys³⁶-GLP-1(7-36)amid; Arg^{26,34}-GLP-1(7-36)amid; Arg^{28,34}Lys⁴⁰-GLP-1(7-36)amid; Arg²⁶Lys³⁶-GLP-1(7-36)amid; Arg³⁴Lys³⁶-GLP-1(7-36)amid; Gly⁸-GLP-1(7-36)amid; Val⁸-GLP-1(7-36)amid, Met⁸-GLP-1(7-36)amid; Gly⁸Asp²²-GLP-1(7-36)amid; Gly⁸Glu²²His³⁷-GLP-1(7-36)amid; Val⁸Asp²²-GLP-1(7-36)amid; Met⁸Asp²²-GLP-1(7-36)amid, Gly⁸Glu²²-GLP-1(7-36)amid; Val⁸Glu²²-GLP-1(7-36)amid; Met⁸Glu²²-GLP-1(7-36)amid; Gly⁸Lys²²-GLP-1(7-36)amid; Val⁸Lys²²-GLP-1(7-36)amid; Met⁸Lys²²-GLP-1(7-36)amid; Gly⁸His²²His³⁷GLP-1(7-36)amid; Gly⁸Arg²²-GLP-1(7-36)amid; Val⁸Arg²²-GLP-1(7-36)amid; Met⁸Arg²²-GLP-1(7-36)amid; Gly⁸His²²-GLP-1(7-36)amid; Val⁸His²²-GLP-1(7-36)amid; Met⁸His²²-GLP-1(7-36)amid; His³⁷-GLP-1(7-36)amid; Val⁸Arg²²His³⁷-GLP-1(7-36)amid; Met⁸Arg²²His³⁷-GLP-1(7-36)amid; Gly⁸His³⁷-GLP-1(7-36)amid; Val⁸His³⁷-GLP-1(7-36)amid; Met⁸His³⁷-GLP-1(7-36)amid; Gly⁸Asp²²His³⁷-GLP-1(7-36)amid; Val⁸Asp²²His³⁷-GLP-1(7-36)amid; Met⁸Asp²²His³⁷-GLP-1(7-36)amid; Val⁸Glu²²His³⁷-GLP-1(7-36)amid; Met⁸Glu²²His³⁷-GLP-1(7-36)amid; Gly⁸Lys²²His³⁷-GLP-1(7-36)amid; Val⁸Lys²²His³⁷-GLP-1(7-36)amid; Met⁸Lys²²His³⁷-GLP-1(7-36)amid; Gly⁸Arg²²His³⁷-GLP-1(7-36)amid; Val⁸His²²His³⁷-GLP-1(7-36)amid und Met⁸His²²His³⁷-GLP-1(7-36)amid.

21. Verfahren nach einem der Ansprüche 1-18, wobei es sich bei dem Peptid um ein GLP-2-Analogon handelt, das ausgewählt ist aus der Gruppe, bestehend aus Lys²⁰GLP-2(1-33); Lys²⁰Arg³⁰GLP-2(1-33); Arg³⁰Lys³⁴GLP-2(1-34); Arg³⁰Lys³⁵GLP-2(1-35); Arg^{30,35}Lys²⁰GLP-2(1-35) und Arg³⁵GLP-2(1-35).

22. Verfahren nach einem der Ansprüche 118. wobei es sich bei dem Peptid um ein Insulinanalogon handelt, bei welchem es sich um des(B30)-Humaninsulin handelt.

23. Verfahren nach einem der vorangehenden Ansprüche, wobei es sich bei dem Peptid um einen GLP-Agonisten handelt.

24. Verfahren nach einem der vorangehenden Ansprüche, wobei das Peptid ausgewählt ist aus der Gruppe, bestehend aus Exendin-3, Exendin-4, Arg³⁴-GLP-1(7-37); Gly⁸-GLP-1(7-36)amid; Gly⁸-GLP-1(7-37); Val⁸-GLP-1(7-36)amid; Val⁸-GLP-1(7-37); Val⁸Asp²²-GLP-1(7-36)amid; Val⁸Asp²²-GLP-1(7-37); Val⁸Glu²²-GLP-1(7-36)amid; Val⁸Glu²²-GLP-1(7-37); Val⁸Lys²²-GLP-1(7-36)amid; Val⁸Lys²²-GLP-1(7-37); Val⁸Arg²²-GLP-1(7-36)amid; Val⁸Arg²²-GLP-1(7-37); Val⁸His²²-GLP-1(7-36)amid; Val⁸His²²-GLP-1(7-37) und des(B30)-Humaninsulin.

25. Verfahren nach Anspruch 19, wobei es sich bei dem Peptid um HGEGTFTSDLSKQMEEEAVRLFIEWLKNGGPSSGAPPSKKKKKK-NH2 (ZP-10) handelt.

26. Verfahren nach einem der Ansprüche 1-14, wobei das Reaktionsgemisch in Schritt a) einen Puffer umfasst, der dazu geeignet ist, während der Umsetzung einen im Wesentlichen konstanten pH-Wert aufrecht zu halten.

27. Verfahren nach einem der vorangehenden Ansprüche, wobei es sich bei dem Peptid um kein Insulinpeptid handelt.

## Revendications

1. Procédé de production d'un peptide N-acylé, ledit procédé comprenant les étapes consistant
a) à faire réagir le peptide ayant au moins un groupe amino libre avec un agent d'acylation de formule générale I 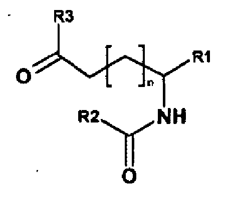 dans laquelle
n vaut 0-8, ;
R¹ est COOR⁴ ;
R² est un fragment lipophile ;
R³, avec le groupe carbonyle auquel R³ est fixé, désigne un ester réactif ou un ester de N-hydroxyimide réactif ; et
R⁴ est choisi parmi l'atome d'hydrogène, les groupes alkyle en C₁₋₁₂ et le groupe benzyle,
dans des conditions basiques en mélange aqueux ;
b) si R⁴ n'est pas un atome d'hydrogène, à saponifier le groupe ester peptidique acylé (COOR⁴) dans des conditions basiques ;
c) à isoler le peptide N-acylé, **caractérisé en ce que** ledit mélange aqueux de l'étape a) contient moins de 80 % p/p d'un solvant polaire aprotique.

2. Procédé selon la revendication 1, dans lequel ladite réaction de l'étape a) a lieu dans un mélange aqueux contenant moins de 5 % p/p d'un solvant polaire aprotique, de préférence moins de 3 % p/p d'un solvant polaire aprotique.

3. Procédé selon l'une quelconque des revendications 1-2, dans lequel l'agent d'acylation est ajouté au mélange réactionnel sous forme d'un solide.

4. Procédé selon l'une quelconque des revendications 1-2, dans lequel ladite réaction de l'étape a) a lieu en présence d'un solvant polaire aprotique, et ledit solvant polaire aprotique est choisi dans le groupe consistant en la N-méthyl-2-pyrrolidone, le tétrahydrofuranne et le diméthylsulfoxyde.

5. Procédé selon la revendication 4, dans lequel la totalité du solvant organique aprotique est ajoutée au mélange réactionnel sous forme d'un solvant pour l'agent d'acylation.

6. Procédé selon l'une quelconque des revendications 4-5, dans lequel l'agent d'acylation est ajouté au mélange réactionnel sous forme d'une solution qui est stabilisée par addition d'un acide.

7. Procédé selon la revendication 6, dans lequel ledit acide est ajouté au solvant polaire aprotique à une concentration de 0,01 % p/p à 1 % p/p, de préférence à une concentration de 0,05 % p/p à 0,5 % p/p.

8. Procédé selon l'une quelconque des revendications 6-7, dans lequel ledit acide est choisi dans le groupe consistant en l'acide sulfurique, l'acide méthanesulfonique et l'acide trifluoracétique.

9. Procédé selon l'une quelconque des revendications 1-3, dans lequel la réaction de l'étape a) a lieu en l'absence d'un solvant polaire aprotique.

10. Procédé selon l'une quelconque des revendications 1-9, dans lequel R⁴ est un atome d'hydrogène.

11. Procédé selon l'une quelconque des revendications 1-9, dans lequel R⁴ est choisi parmi les groupes alkyle en C₁₋₆ et le groupe benzyle.

12. Procédé selon l'une quelconque des revendications 1-11, dans lequel R³, avec le groupe carboxyle auquel R³ est fixé, désigne un ester de N-hydroxy imide réactif.

13. Procédé selon l'une quelconque des revendications 1-9, dans lequel l'ester peptidique acylé est saponifié à un pH compris dans la plage de 10-14, de préférence dans la plage de 9-13.

14. Procédé selon l'une quelconque des revendications 1-13, dans lequel le pH du mélange réactionnel de l'étape a) vaut de 9 à 13, de préférence de 10 à 12 ou d'une manière plus préférée de 11,0 à 11,5.

15. Procédé selon l'une quelconque des revendications 1-14, dans lequel la température du mélange réactionnel de l'étape a) est comprise dans la plage de 0-50°C, de préférence dans la plage de 5-40°C et d'une manière plus préférée dans la plage de 10-30°C.

16. Procédé selon l'une quelconque des revendications précédentes, dans lequel R² est choisi parmi les groupes alkyle en C₃₋₃₉, alcényle en C₃₋₃₉, alcadiényle en C₃₋₃₉ et les résidus stéroïdiens.

17. Procédé selon la revendication 16, dans lequel R²-C(=O)- est choisi dans le groupe consistant en les groupes lithocholoyle et hexadécanoyle.

18. Procédé selon l'une quelconque des revendications précédentes, dans lequel ledit peptide utilisé comme matière de départ pour l'étape a) a une pureté du peptide d'au moins 80 %, d'au moins 90 %, d'au moins 93 %, d'au moins 95 % ou d'au moins 97 %, telle que déterminée par CLHP en phase inversée.

19. Procédé selon l'une quelconque des revendications précédentes, dans lequel ledit peptide est choisi dans le groupe consistant en le GLP-1, l'exendine-4, le GLP-2, le glucagon et l'insuline.

20. Procédé selon l'une quelconque des revendications 1-18, dans lequel ledit peptide est un analogue du GLP-1, qui est choisi dans le groupe consistant en Arg²⁶-GLP-1(7-37); Arg³⁴-GLP-1(7-37); Lys³⁶-GLP-1(7-37); Arg^{26,34}Lys³⁶-GLP-1(7-37); Arg^{26,34}Lys³⁸GLP-1(7-38); Arg^{26,34}Lys³⁹-GLP-1(7-39); Arg^{26,34}Lys⁴⁰-GLP-1(7-40); Arg³⁴Lys-³⁶-GLP-1(7-37); Arg²⁶Lys³⁹-GLP-1(7-39); Arg³⁴Lys⁴⁰-GLP-1(7-40); Arg^{26,34}Lys^{36,39}-GLP-1(7-39); Arg^{26,34}Lys^{36,40}-GLP-1(7-40); Gly⁶Arg²⁶-GLP-1(7-37); Gly⁸Arg³⁴-GLP-1(7-37); Gly⁸Lys³⁶-GLP-1(7-37); Gly⁸Arg^{26,34}Lys³⁶-GLP-1(7-37); Gly⁸Arg^{26,34}Lys³⁹-GLP-1(7-39); Gly⁸Arg^{26,34}Lys⁴⁰-GLP-1(7-40); Gly⁸Arg²⁶Lys³⁶-GLP-1(7-37); Gly⁸Arg³⁴Lys³⁶-GLP-1(7-37); Lys³⁶-GLP-1(7-37); Arg^{26,34}Lys³⁶-GLP-1(7-37); Arg^{26,34}-GLP-1 (7-37); Arg^{26,34}Lys⁴⁰-GLP-1(7-37); Arg²⁶Lys³⁶-GLP-1(7-37); Arg³⁴Lys³⁶-GLP-1(7-37); Val⁸Arg²²-GLP-1(7-37); Met⁸Arg²²-GLP-1(7-37);Gly⁸His²²-GLP-1(7-37); Val⁸His²²-GLP-1(7-37); Met⁸His²²-GLP-1(7-37);His³⁷-GLP-1(7-37); Gly⁸-GLP-1(7-37); Val⁸-GLP-1(7-37); Met⁸-GLP-1(7-37);Gly⁸Asp²²-GLP-1(7-37); Val⁸Asp²²-GLP-1(7-37); Met⁸Asp²²-GLP-1(7-37);Gly⁸Glu²²-GLP-1(7-37); Val⁸Glu²²-GLP-1(7-37); Met⁸Glu²²-GLP-1(7-37); Gly⁸Lys²²-GLP-1(7-37); Val⁸Lys²²-GLP-1(7-37); Met⁸Lys²²-GLP-1(7-37); Gly⁸Arg²²-GLP-1(7-37); Val⁸Lys²²His³⁷-GLP-1(7-37); Gly⁸Glu²²His³⁷-GLP-1(7-37); Val⁸Glu²²His³⁷-GLP-1(7-37); Met⁸Glu²²His³⁷-GLP-1(7-37);Gly⁸Lys²² His³⁷-GLP-1(7-37); Met⁸Lys²²His³⁷-GLP-1(7-37);Gly⁸Arg²²His³⁷-GLP-1(7-37); Val⁸Arg²²His³⁷-GLP-1(7-37); Met⁸Arg²²His³⁷-GLP-1(7-37); Gly⁸His²²His³⁷-GLP-1(7-37); Val⁸His²²His³⁷-GLP-1(7-37); Met⁸His²²His³⁷-GLP-1(7-37); Gly⁸His³⁷-GLP-1(7-37); Val⁸His³⁷-GLP-1(7-37); Met⁸His³⁷-GLP-1(7-37);Gly⁸Asp²² His³⁷-GLP-1(7-37); Val⁸Asp²²His³⁷-GLP-1(7-37); Met⁸Asp²²His³⁷-GLP-1(7-37); Arg²⁶-GLP-1(7-36)-amide; Arg³⁴ -GLP-1(7-36)-amide; Lys³⁶-GLP-1(7-36)-amide; Arg^{26,34}Lys³⁶-GLP-1(7-36)-amide; Arg^{26,34}-GLP-1(7-36)-amide; Arg^{26,34}Lys⁴⁰-GLP-1(7-36)-amide; Arg²⁶Lys³⁶-GLP-1(7-36)-amide; Arg³⁴Lys³⁶-GLP-1(7-36)-amide; Gly⁸-GLP-1(7-36)-amide; Val⁸-GLP-1 (7-36)-amide; Met⁸-GLP-1(7-36)-amide;Gly⁸Asp²²-GLP-1(7-36)-amide; Gly⁸Glu²²His³⁷-GLP-1(7-36)-amide; Val⁸Asp²²-GLP-1(7-36)-amide; Met⁸Asp²²-GLP-1(7-36)-amide;Gly⁸Glu²²-GLP-1(7-36)-amide; Val⁸Glu²²-GLP-1 (7-36)-amide; Met⁸Glu²²-GLP-1 (7-36)-amide; Gly⁸Lys²²-GLP-1(7-36)-amide; Val⁸Lys²²-GLP-1 (7-36)-amide; Met⁸Lys²²-GLP-1(7-36)-amide; Gly⁸His²²His³⁷-GLP-1(7-36)-amide; Gly⁸Arg²²-GLP-1(7-36)-amide; Val⁸Arg²²-GLP-1(7-36)-amide; Met⁸Arg²²-GLP-1(7-36)-amide;Gly⁸His²²-GLP-1(7-36)-amide; Val⁸His²²-GLP-1(7-36)-amide; Met⁸His²²-GLP-1(7-36)-amide; His³⁷-GLP-1(7-36)-amide; Val⁸Arg²²His³⁷-GLP-1 (7-36)-amide; Met⁸Arg²²His³⁷-GLP-1(7-36)-amide; Gly⁸His³⁷-GLP-1 (7-36)-amide; Val⁸His³⁷-GLP-1 (7-36)-amide; Met⁸His³⁷-GLP-1 (7-36)-amide;Gly⁸Asp²² His³⁷-GLP-1(7-36)-amide; Val⁸Asp²²His³⁷-GLP-1(7-36)-amide; Met⁸Asp²² His³⁷-GLP-1 (7-36)-amide; Val⁸Glu²²His³⁷-GLP-1(7-36)-amide; Met⁸Glu²²His³⁷-GLP-1(7-36)-amide;Gly⁸Lys²² His³⁷-GLP-1 (7-36)-amide; Val⁸Lys²²His³⁷-GLP-1(7-36)-amide; Met⁸Lys²²His³⁷-GLP-1 (7-36)-amide ; Gly⁸Arg²²His³⁷-GLP-1(7-36)-amide Val⁸His²²His³⁷-GLP-1(7-36)-amide ; et Met⁸His²²His³⁷-GLP-1(7-36)-amide.

21. Procédé selon l'une quelconque des revendications 1-18, dans lequel ledit peptide est un analogue du GLP-2 qui est choisi dans le groupe consistant en les analogues suivants : Lys²⁰GLP-2(1-33) ; Lys²⁰Arg³⁰GLP-2(1-33); Arg³⁰Lys³⁴GLP-2(1-34); Arg³⁰Lys³⁵GLP-2(1-35); Arg^{30,35}Lys²⁰GLP-2(1-35) ; et Arg³⁵GLP-2(1-35).

22. Procédé selon l'une quelconque des revendications 1-18, dans lequel ledit peptide est un analogue de l'insuline, qui est une insuline des (B30)-humaine.

23. Procédé selon l'une quelconque des revendications précédentes, dans lequel ledit peptide est un agoniste du GLP-1.

24. Procédé selon l'une quelconque des revendications précédentes, dans lequel ledit peptide est choisi dans le groupe consistant en l'exendine-3, l'exendine-4, Arg³⁴-GLP-1 (7-37), Gly⁸-GLP-1(7-36)-amide, Gly⁸-GLP-1 (7-37), Val⁸-GLP-1 (7-36)-amide, Val⁸-GLP-1(7-37), Val⁸Asp²²-GLP-1 (7-36)-amide, Val⁸Asp²²-GLP-1(7-37), Val⁸Glu²²-GLP-1 (7-36)-amide, Val⁸Glu²²-GLP-1(7-37). Val⁸ Lys²²-GLP-1(7-36)-amide, Val⁸Lys²²-GLP-1(7-37), Val⁸Arg²²-GLP-1(7-36)-amide, Val⁸Arg²²-GLP-1 (7-37), Val⁸His²²-GLP-1 (7-36)-amide, Val⁸His²²-GLP-1(7-36), et l'insuline des(B30)-humaine.

25. Procédé selon la revendication 19, dans lequel ledit peptide est HGEGTFTSDLSKQMEEEAVRLFIEWLKNGGPSSGAPPSKKKKKK-NH2 (ZP-10).

26. Procédé selon l'une quelconque des revendications 1-14, dans lequel le mélange réactionnel de l'étape a) comprend un tampon qui convient au maintien d'un pH essentiellement constant pendant la réaction.

27. Procédé selon l'une quelconque des revendications précédentes, dans lequel ledit peptide n'est pas un peptide insulinique.
